# EUROPEAN PATENT APPLICATION

(11) **EP 3 031 414 A1**
(43) Date of publication of application: **15.06.2016**
(21) Application number: 15184961.9
(22) Date of filing: 11.09.2015
(51) Int. Cl.: A61B 17/322, A61F 2/10

(54) **MECHANIZED PUNCH FOR MAKING CYLINDRICAL EXCISION OF HAIR FOLLICLES**

(30) Priority: 10.12.2014 TW 103142954
(71) Applicant: Shiao, Tseng-Kuo, Taipei (TW)
(72) Inventor: Shiao, Tseng-Kuo, Taipei (TW)
(74) Representative: Lang, Christian

(57) **Abstract**

A mechanized punch for making cylindrical excision of hair follicles (10) is connected to an electronic instrument (60) and includes a tubular element (11) and a rectilinearly advancing rotary mechanism (20). The rectilinearly advancing rotary mechanism (20) includes a circle-cutting device (21) and a rectilinear movement module (40), in which the circle-cutting device (21) comprises a transmission shaft (22) having a first end connected with a drive element (25) and a second end connected with a chuck (23) so that the chuck (23) is allowed to clamp tight a tubular punch (24). When donor skin (52) is contacted by the tubular punch (24), the electronic instrument (60) actuates the drive element (25) to drive the transmission shaft (22) and the tubular punch (24) for rotation while the linear actuator (44) in the rectilinear movement module (40) drives the circle-cutting device (21) in a rectilinear forward motion until a cylindrical cut (53) of predetermined depth is made on the donor skin (52) by the punch (24).

## Description

### BACKGROUND OF THE INVENTION

### Field of the Invention

The present invention relates to a novel mechanized punch for making cylindrical excision of hair follicles and other skin appendages or lesions on the skin.

### Description of the Related Art

Hair loss, or androgenic alopecia, affects up to 70% of men and 40% of women. While many people do not consider hair loss a disease, hair loss sufferers often lose their self-confidence and have trouble developing healthy interpersonal relationship. In severe cases, those afflicted may even become suicidal. Hair loss, therefore, should not to be taken lightly.

Clinically, in addition to appropriate medical therapies, the appearance of hair loss can also be improved surgically by various surgical procedures, collectively known as hair transplant. The objective of a hair transplant is to move hair follicles from one area of the skin, typically the posterior and lateral aspects of the scalp, hereon refer as the donor, to another area of the skin that is deficient of hair, typically the top of the scalp, hereon refer as the recipient, where these relocated hair serve to improve one's look. To achieve the most natural appearance, hair follicles are often moved as follicular units, the natural groupings of hair follicles. Each follicular unit typically contains 1 to 4 hairs and falls within a cylindrical volume about 1 mm in diameter and 4 to 6 mm deep.

Follicular Unit Extraction (FUE) is a type of hair transplant gaining popularity in recent years. In FUE, the surgeon first cuts the cylindrical border of a follicular unit with a tubular punch, and then grasps the excised follicular unit with an instrument such as forceps to pull the follicular unit out of the donor. Current technology employs a fixed or rotating tubular punch that the surgeon manually advances into the donor skin until it reaches a depth (approximately 2-5mm) that allows easy extraction of the excised follicular unit.

Manually advancing a circle cutting tubular punch in hope to cut a perfect cylinder within which the follicular unit resides often encounters the following difficulties and issues. First of all, since the hand holding the punch hinges at the wrist, the hand naturally moves the punch in an arc. This non-linear movement easily leads to full or partial transection of hair follicles during the cylinder cutting process, resulting in extraction of incomplete hair follicles which may not grow after transplanted.

Secondly, when hair exits the skin at a more acute angle, because of simple geometry, the circle-cutting punch must advance a longer distance in order to penetrate the dermis which is necessary to facilitate easy extraction of follicular units later. The shallow angle and the long distance of travel combination makes it particularly difficult to maintain the necessary linear path for cutting a perfect cylinder, resulting in higher transection rate of hair follicles.

Thirdly, higher transection rate means it takes more attempts and time to obtain the desired number of good grafts for transplant.

Lastly, the surgeon must perform the same movement thousands of times for each hair transplant, which can lead to occupational repetitive stress injury over time.

### BRIEF SUMMARY OF THE INVENTION

The primary objective of the present invention is to provide a mechanized punch for making cylindrical excision of hair follicles. Unlike current devices, manual or mechanized with a rotating tubular punch, that only cut a 2-dimensional circle; this invention cuts a perfect mechanized punch for making cylindrical excision of hair follicles by simultaneously rotating a tubular punch and advancing it rectilinearly. The intact follicular unit contained within the mechanized punch for making cylindrical excision of hair follicles can then be extracted after the mechanized punch for making cylindrical excision of hair follicles is pulled from the donor skin.

A secondary objective of the present invention is to provide a mechanized punch for making cylindrical excision of hair follicles that can be connected to an electronic instrument to precisely control the depth, the advancement speed and the rotational speed of its attached tubular punch.

To attain the above described objectives, a mechanized punch for making cylindrical excision of hair follicles of the invention comprises a tubular element that contains a rectilinearly advancing rotary mechanism. The rectilinearly advancing rotary mechanism comprises a two-dimensional circle-cutting device and a rectilinear movement module, in which the circle cutting device comprises a transmission shaft having a first end connected with a drive element and a second end connected with a chuck so that the chuck can clamp tight a tubular punch. The rectilinear movement module comprises a support plate that is fixed to the tubular element and carries a connection terminal, a touch sensor circuitry and a linear actuator so that the connection terminal, the touch sensor circuitry and the linear actuator are allowed to electrically connect to an electronic instrument, and the drive element from the circle-cutting device is both electrically and mechanically connected to the rectilinear movement module. The touch sensor circuitry sends a signal to the electronic instrument when the tubular punch contacts the donor skin (typically the scalp). The electronic instrument, based on operator designated parameters, actuates the drive element from the circle-cutting device that drives the transmission shaft for rotation which in turn drives the tubular punch for rotation, and simultaneously signals the linear actuator from the rectilinear movement module to drive the circle-cutting device in a rectilinear forward motion to cut a predetermined depth on the donor skin. The circle-cutting device is then retracted by the rectilinear movement module, so that the follicular unit cut by the mechanized punch for making cylindrical excision of hair follicles on the donor skin can be extracted once the cylinder-cutting operation is completed.

A detailed description is given in the following embodiments with reference to the accompanying drawings.

### BRIEF DESCRIPTION OF THE DRAWINGS

The present invention can be better explained by the subsequent detailed descriptions and references made to the accompanying drawings, wherein:
Fig. 1 is a perspective view of the mechanized punch for making cylindrical excision of hair follicles of the invention;
Fig. 2 is a partially-sectioned perspective view of the mechanized punch for making cylindrical excision of hair follicles of the invention;
Fig. 3 is an exploded view of the tubular element in the mechanized punch for making cylindrical excision of hair follicles of this invention;
Fig. 4 is a perspective view of a rectilinearly advancing rotary mechanism in the mechanized punch for making cylindrical excision of hair follicles of this invention, in which a chuck and a tubular punch are not assembled with a transmission shaft;
Fig. 5 is a bottom perspective view of a rectilinearly advancing rotary mechanism in the mechanized punch for making cylindrical excision of hair follicles of this invention;
Fig. 6 is a longitudinal sectional view of the mechanized punch for making cylindrical excision of hair follicles of the invention;
Figs. 7 and 8 are bottom partially-sectioned views, illustrating a rectilinearly advancing rotary mechanism be moved from a first position to a second position;
Figs. 9, 10, 11 and 12 are schematic views illustrating the follicular unit excising action of a mechanized punch for making cylindrical excision of hair follicles on a donor skin.

### DETAILED DESCRIPTION OF THE INVENTION

The following description is of the best-contemplated mode of carrying out the invention. This description is made for the purpose of illustrating the general principles of the invention and should not be taken in a limiting sense. The scope of the invention is best determined by reference to the appended claims.

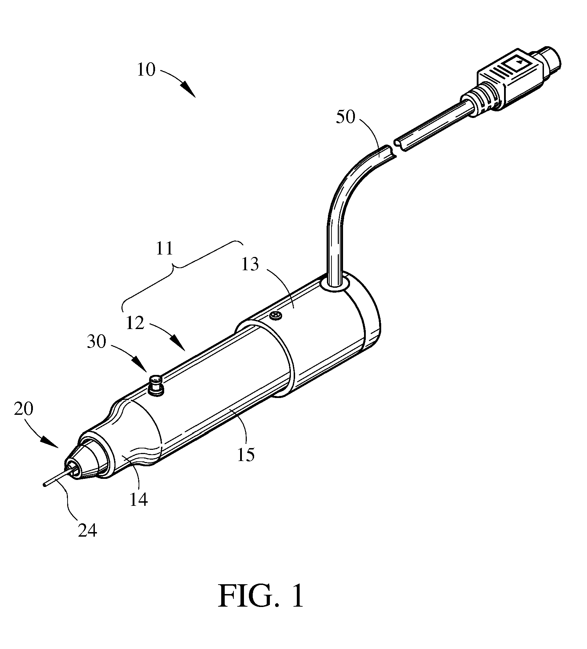

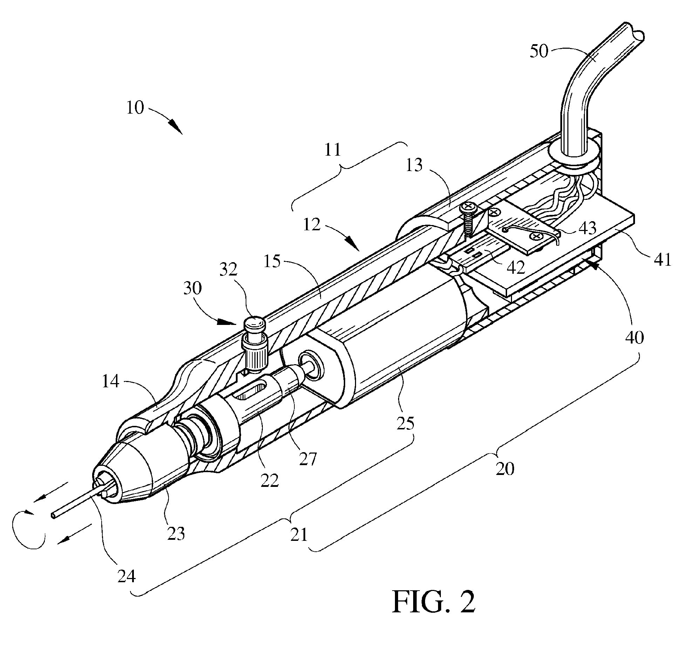

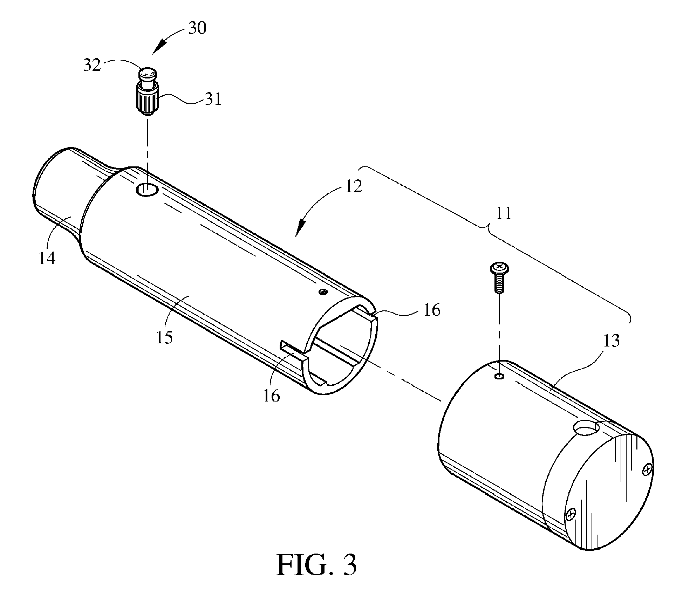

Referring to Figs. 1 and 2, the mechanized punch for making cylindrical excision of hair follicles 10 of the invention comprises a tubular element 11 and a rectilinear advancing rotary mechanism 20 installed in the tubular element 11. As shown in Fig. 3, the tubular element 11 comprises a handheld tube 12 and a cap 13, in which a partial length of a distal end of the handheld tube 12 is extended into the cap 13, and the handheld tube 12 and the cap 13 are connected with each other via a screw or other fixation methods. The handheld tube 12 comprises a first tubular portion 14 and a second tubular portion 15 integrally formed (or separately formed then assembled) with the first tubular portion 14. The diameter of the first tubular portion 14 is substantially less than that of the second tubular portion 15. The handheld tube 12 has an opening end or a top pipe wall that is formed with two downward corresponding positioning grooves 16.

The tubular element 11 further comprises a pressing element 30 mounted on the second tubular end portion 15 of the handheld tube 12, an outer ring 31 installed in a hole of the second tubular end portion 15 of the handheld tube 12, and a telescopic rod 32 disposed in the outer ring 31. The telescopic rod 32 is moves inwards of the second tubular end portion 15 of the handheld tube 12 for a predetermined distance when a pressing force is applied thereon and the telescopic rod 32 is automatically returned when the pressing force is released therefrom.

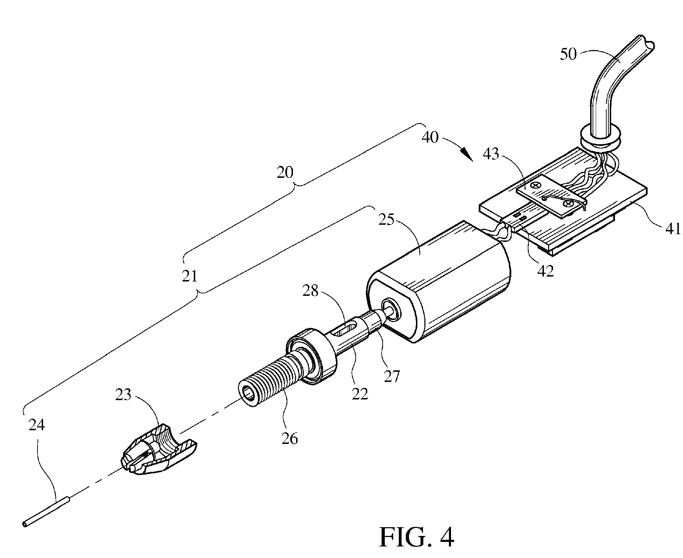

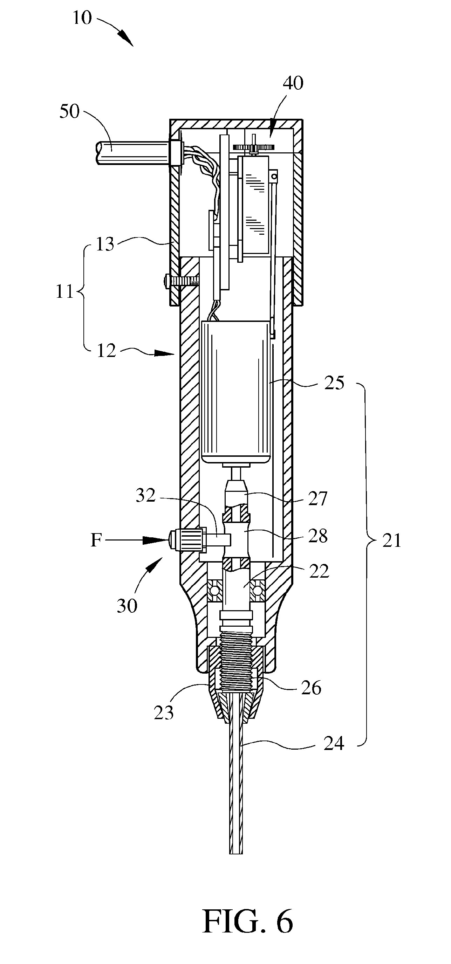

As shown in Fig. 4, the rectilinearly advancing rotary mechanism 20 comprises a circle-cutting device 21 and a rectilinear movement module 40. The circle-cutting device 21 comprises a transmission shaft 22, a chuck 23, a tubular punch 24 and a drive element 25 (e.g., a motor or other drivers). The transmission shaft 22, which has a first end connected with the chuck 23 and a second end connected with the drive element 25, comprises a threaded end 26 provided for lock-in and connection of the chuck 23, a connection end 27 connected to an output shaft of the drive element 25 to drive the transmission shaft 22 for rotation, and an elongated slot 28 disposed next to the connection end 27. When the telescopic rod 32 is pressed by an acting force F, a lower part of the telescopic rod 32 is inserted into the elongated slot 28 of the transmission shaft 22 (as shown in Fig. 6), thereby preventing the transmission shaft 22 from being rotated and allowing the rotation of the chuck 23 around the threaded end 26 of the transmission shaft 22, thereby clamping down (see Fig. 1) or loosening (see Fig. 4) the tubular punch. The tubular punch 24 can be clamped by the chuck 23 when the chuck 23 is fully screwed on the threaded end 26 of the transmission shaft 22. Because the chuck 23 is already in use as a commercial product, the description connected therewith is omitted here.

The rectilinear movement module 40 comprises a support plate 41 that is fixed to the tubular element 11, i.e., slotted in the positioning grooves 16 of the second tubular end portion 15 of the handheld tube 12 and partially located in the cap 13. The support plate 41 of the rectilinear movement module 40 is utilized to carry a connection terminal 42 and a touch sensor circuitry 43 that are electrically connect to an electronic instrument (not shown in FIGs.). A transmission cable 50 arranged between the electronic instrument and the connection terminal 42/the touch sensing circuitry 43 is utilized for transmitting electrical signal. The drive element 25 is electrically connected to the connection terminal 42. The electronic instrument is capable of providing independent driven processes, such as forward/backward distance/speed and rotational speed of the rectilinearly advancing rotary mechanism 20. Because the touch sensor circuitry 43 is already in use as a commercial product, the description connected therewith is omitted here. The tubular punch 24 is used of detecting external signals by the touch sensor circuitry 43. When a conductive object is contacted by the punch 24, the touch sensor circuitry 43 sends a signal to the electronic instrument via the transmission cable 50 which in turn actuates the drive element 25 based on operator designated parameters on rotational speed, thus to drive the transmission shaft 22 for rotation which simultaneously drive the punch 24 for rotation. Accordingly, the electronic instrument simultaneously use the operator designated parameters to control the rectilinear advancing distance and the forwarding speed of the punch 24 via the mechanism described below.

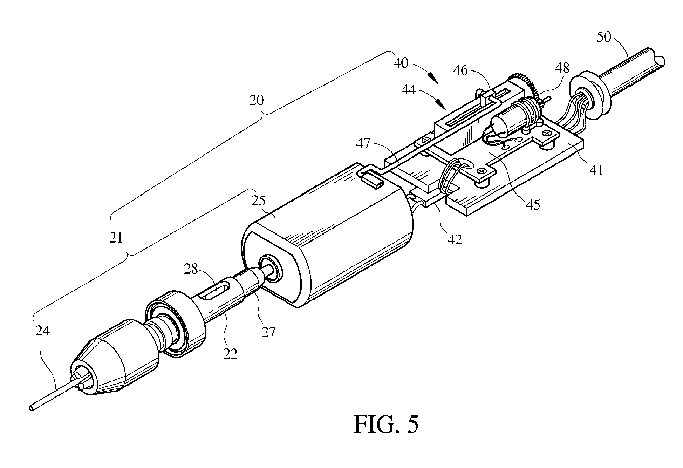

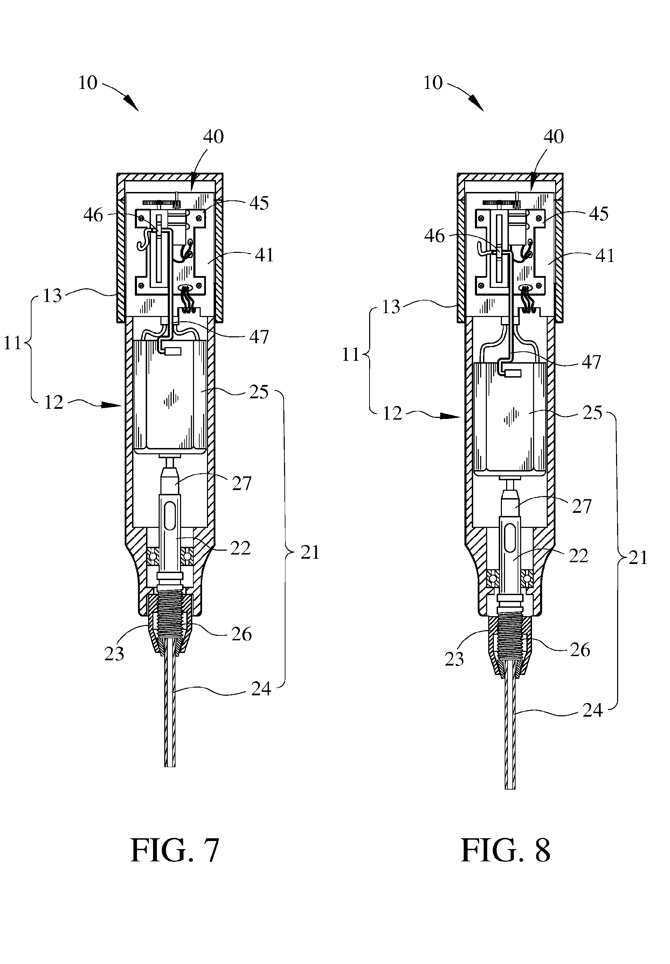

As shown in Figs. 4 and 5, the rectilinear movement module 40 further comprises a linear actuator 44 (e.g., a linear servo motor or a micro servo motor) that is installed on one surface of the support plate 41. Because the linear actuator 44 is already in use as a commercial product, the description connected therewith is omitted here. The linear actuator 44 comprises a printed circuit board 45 electrically connected with the transmission cable 50, a slide block 46, and a conductive linkage 47 that is disposed between the slide block 46 and the drive element 25 to form a linked-movement relationship therebetween and to form electrical connection of the conductive linkage 47 and the touch sensor circuitry 43. With the gear structure of the slide block 46 to be driven by a motor 48 of the linear actuator 44, the slide block 46 is allowed to be moved forward and backward in a rectilinear direction. For example, the slide block 46 is moved from a first position (see Fig. 7) to a second position (see Fig. 8) in the rectilinear direction so that the circle-cutting device 21 is driven to move in the rectilinear direction to push forward the two-dimensional circle-cutting tubular punch 24 for a predetermined distance, cutting a perfect three-dimensional cylinder, and then the slide block 46 is returned from the second position (see Fig. 8) to the first position (see Fig. 7) so that the tubular punch 24 is retracted to the initial position thereof.

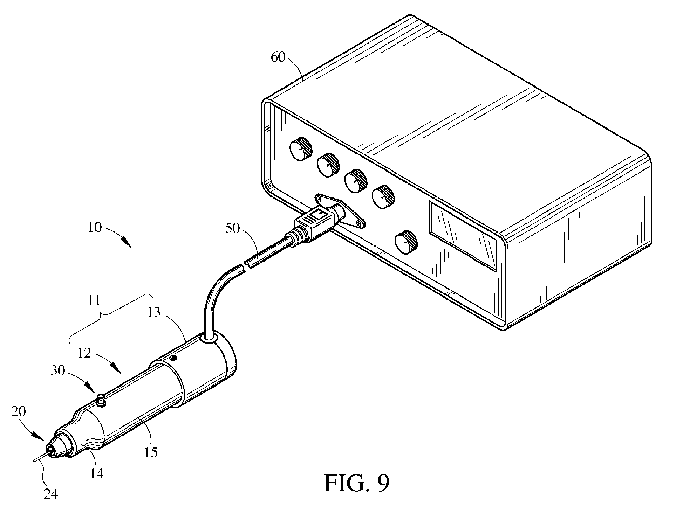

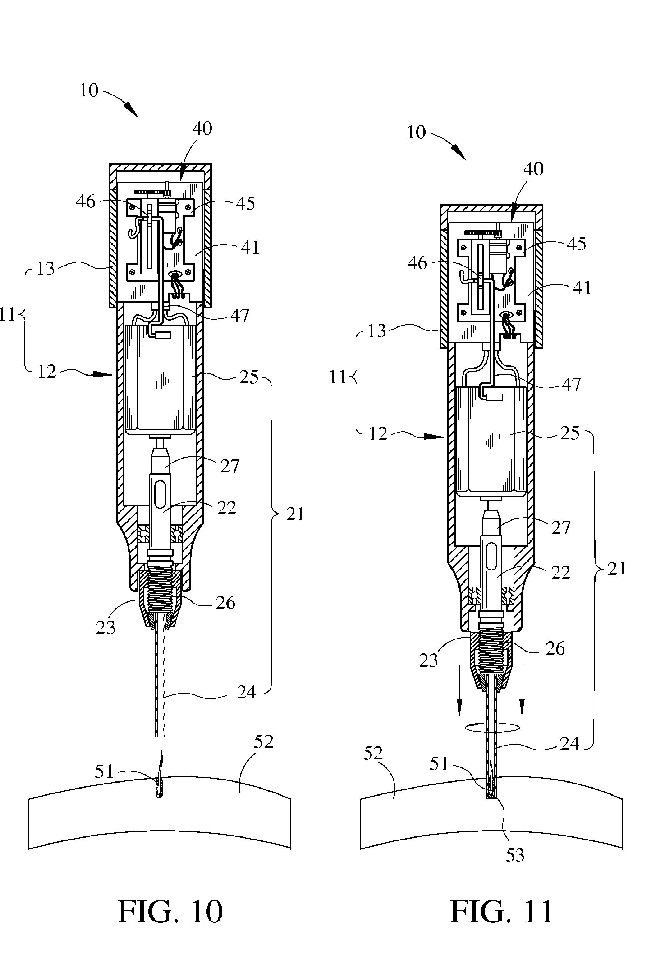

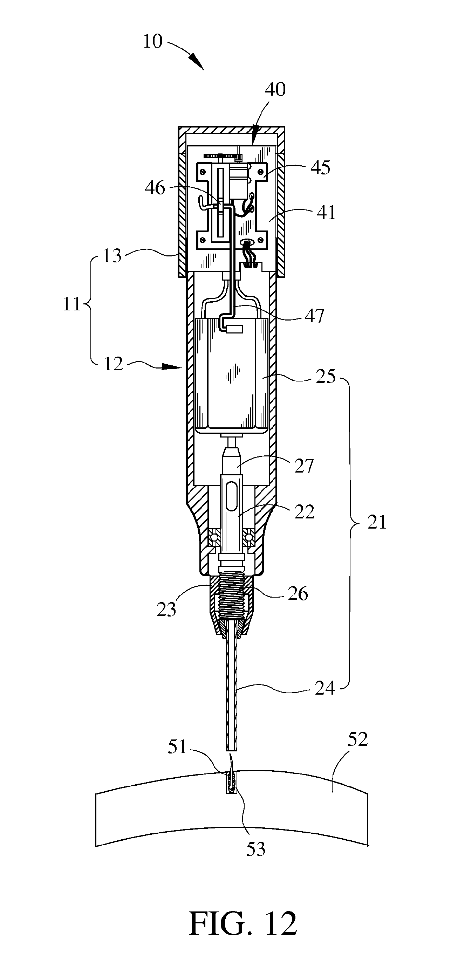

Figs. 9, 10, 11 and 12 illustrate the operation steps of the mechanized punch for making cylindrical excision of hair follicles 10 on a hair follicle from donor skin (typically scalp) 52. As shown in Fig. 9, the mechanized punch for making cylindrical excision of hair follicles 10 is firstly electrically connected to an electronic instrument 60. As shown in Fig. 10, in the operation of the mechanized punch for making cylindrical excision of hair follicles 10, the tubular punch 24 is moved forward in the growth direction (taking a hair as a guidance) of the follicular unit 51. When the donor skin 52 is contacted by the tubular punch 24, the touch sensing circuitry 43 sends a signal to the electronic instrument 60 via transmission cable 50, a control circuit of the electronic instrument 60 uses the operator designated parameters to actuate the drive element 25 to drive the transmission shaft 22 for rotation and simultaneously to drive the tubular punch 24 for rotation via the transmission shaft 22, and the electronic instrument 60 at the same time transmits a signal to the printed circuit board 45 of the linear actuator 44 to command the motor 48 of the linear actuator 44 for driving the slide block 46 rectilinearly and forwardly move for a predetermined distance which simultaneously driving the rectilinearly advancing rotary mechanism 20 to move toward the donor skin 52, thereby pushing the rotating tubular punch 24 into the donor skin 52. As shown in Fig. 11, the rotating tubular punch 24 is pushed into the donor skin 52 until a cylindrical cut 53 having a predetermined depth is made on the donor skin 52 by the tubular punch 24. As shown in Fig. 12, the tubular punch 24 is then allowed to be returned to its initial position thereof, so that the hair follicle on the donor skin 5 2 is ready to be extracted at the completion of the cylinder-cutting operation.

In conclusion, with the mechanized punch for making cylindrical excision of hair follicles 10 of the invention capable of simultaneously performing the circular cutting operation and the rectilinear advancing movement, thereby cutting a perfect cylinder around the hair follicle to facilitate extraction of intact hair follicles. Furthermore, with the mechanized punch for making cylindrical excision of hair follicles 10 connected to the electronic instrument 60, the advancing depth, the forwarding speed and the rotational speed of the tubular punch 24 can be precisely controlled.

While the invention has been described by way of example and in terms of the preferred embodiments, it is to be understood that the invention is not limited to the one disclosed embodiment. To the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. A mechanized punch for making cylindrical excision of hair follicles (10), which is mechanically and electronically connected to an electronic instrument (60), and comprises:
a tubular element (11); and
a rectilinearly advancing rotary mechanism (20) which comprises a two-dimensional circle-cutting device (21) and a rectilinear movement module (40), in which the circle-cutting device (21) comprises a transmission shaft (22) having a first end connected with a drive element (25) and a second end connected with a chuck (23) so that the chuck (23) is capable of clamping tightly a tubular punch (24), and the rectilinear movement module (40) comprises a support plate (41) that is fixed to the tubular element (11) and carries a connection terminal (42), a touch sensor circuitry (43) and a linear actuator (44) all of which are electrically connected to the electronic instrument (60), and the drive element (25) is electrically and mechanically connected to the rectilinear movement module (40), the touch sensor circuitry (43) sends a signal to the electronic instrument (60) when the tubular punch (24) contacts the donor skin (52), and the electronic instrument (60), based on predetermined parameters, actuates the drive element (25) to drive the transmission shaft (22) for rotation which in turn drive the tubular punch (24) for rotation, and simultaneously send signals to the linear actuator (44) to drive the circle-cutting device (21) in a rectilinear forward motion until a mechanized punch for making cylindrical excision of hair follicles (10) of a predetermined depth is made on the donor skin (52) by the punch, then the circle-cutting device (21) is retracted by the rectilinear movement module (40), so that the follicular unit (51) cut by the mechanized punch for making cylindrical excision of hair follicles (10) can be extracted once the cylinder-cutting operation is completed.

2. The mechanized punch for making cylindrical excision of hair follicles (10) as claimed in claim 1, wherein the tubular element (11) comprises a handheld tube (12) and a cap (13), in which the cap (13) end of handheld tube (12) has two slots that are open at one end, together with correspondingly positioning grooves (16) inside the cap (13), interlock with the support plate (41) to form one cohesive unit.

3. The mechanized punch for making cylindrical excision of hair follicles (10) as claimed in claim 1, wherein a conductive linkage (47) connecting the sliding block (46) of the linear actuator (44) from the rectilinear movement module (40) to the driving element (25) from the circle cutting device (21) form both a linked movement relationship therebetween and an electrical touch sensing pathway from the punch (24) to the touch sensor circuitry (43)
